# EUROPEAN PATENT APPLICATION

(11) **EP 1 712 636 A1**
(43) Date of publication of application: **18.10.2006**
(21) Application number: 05719057.1
(22) Date of filing: 28.01.2005
(51) Int. Cl.: C12Q 1/02, C12C 1/00, C12C 11/00

(54) **METHOD OF QUICKLY MEASURING FACTOR CAUSING EARLY FLOCCULATION OF YEAST**

(30) Priority: 30.01.2004 JP 2004022532; 30.01.2004 JP 2004022739
(71) Applicant: KIRIN BEER KABUSHIKI KAISHA, Tokyo 104-8288 (JP)
(72) Inventor: OGAWA, Toshiya c/o Kirin Beer Kabushiki Kaisha, Yokohama-shi, Kanagawa 236-0004 (JP); KOIZUMI, Hideki c/o Kirin Beer Kabushiki Kaisha, Yokohama-shi, Knagawa 236-0004 (JP)
(74) Representative: HOFFMANN EITLE
(86) International application number: PCT/JP2005/001199
(87) International publication number: WO 2005/073394

(57) **Abstract**

The present invention provides a method for quickly measuring factors causing early flocculation of yeast contained in brewing materials, and a method for quickly determining early flocculating property of yeast by using the measuring method. Yeast at late logarithmic growth phase or thereafter is prepared, and the yeast is mixed, suspended with water-extracted high-molecular weight fraction of test material sample from oat or malt in a buffer solution, and by measuring the precipitation level of the mixed and suspended yeast, it is possible to measure factors causing early flocculation of yeast contained in brewing materials without performing a fermentation process as in conventional method, in a quite short time period, with a minute amount of sample. The water-extracted high-molecular weight fraction of the test material sample can be easily prepared by extracting brewing materials with water, and then by using ethanol precipitation, or dialysis, ultrafiltration, gel filtration, and the like. Further, precipitation level of yeast can be measured accurately with an easy and simple means such as a measuring method using optical density by using OD600.

## Description

### Technical Field

The present invention relates to a method for quickly measuring factors causing early flocculation of yeast, contained in brewing materials used for manufacturing fermented malt beverages, such as beer, HAPPOSHU (low-malt beer), or whiskey, and to a method for quickly determining early flocculating property of yeast in brewing materials using thereof.

### Background Art

During the brewing of fermented malt beverages or the like such as beer, HAPPOSHU, or whiskey, yeast flocculates and precipitates reasonably at the end of the main fermentation by yeast. After this flocculation and precipitation, yeast can be collected. If yeast does not flocculate and precipitate reasonably at the end of the main fermentation, the recovery level of yeast will be insufficient, and on the contrary, if the flocculation and precipitation are excessive, fermentation will not proceed, thus causing a problem. The flocculation of yeast occurs at the end of fermentation, when extract lessens and yeast mutually forms a clump, and by this flocculation, yeast further precipitates beneath the culture solution. It is reported that the binding of lectin-like protein of yeast cell envelope with mannose sugar chain of yeast mannan is involved in the flocculation of yeast itself (Appl. Microbiol. Biotechnol 23:197-205, 2003).

Thus, during the brewing of fermented malt beverages, in a normal fermentation step, flocculation and precipitation of yeast occurs reasonably at the end of the main fermentation of yeast, when extract of fermentation solution lessens. However, it has been reported that a phenomenon called "early flocculation phenomenon" is observed to this flocculation and precipitation of yeast. This "early flocculation phenomenon" relates to a phenomenon wherein yeast flocculates and precipitates, while assimilable sugar in yeast is still present in the fermentation solution, during the fermentation process of yeast, particularly in the late phase of the fermentation. When yeast flocculates and precipitates by this early flocculation phenomenon, the fermentation process will stop. Therefore, when this phenomenon occurs, fermentation will be insufficient, thus the manufactured products would be below standard, and it would be a significant loss in the brewing of fermented malt beverages and the like.

In order to solve the problem of early flocculation phenomenon of yeast during the fermentation process of the brewing of fermented malt beverages, many studies to elucidate the cause have been made and reported, and as a result, it has been revealed that the early flocculation phenomenon is caused by high-molecular weight acidic polysaccharides included in malt, derived from the material oats. Moreover, it has been revealed that factors inducing the early flocculation phenomenon are, in some case, generated during the oat manufacturing process, while in other cases they are originally present in the material oats (J. Inst. Brew., 97, 359-366, 1991; Journal of Japan Society for Bioscience, Biotechnology and Agrochemistry, 71, 381, 1997; Japanese Laid-Open Patent Application No. 10-179190). Conventionally, in brewing of fermented malt beverages comprising barley as a raw material, in order to avoid the early flocculation phenomenon during the fermentation process, presence or absence of the early flocculating property of malt and barley was confirmed to select and use barley and malt that do not induce early flocculation phenomenon.

In order to confirm the early flocculating property of malt, barley, etc., a method using a fermentation test has been applied as conventional method (K. Morimoto, et. al., Rept. Res. Lab. Kirin Brewery Co., Ltd., 18, 63, 1975). Fermentation test is a test wherein the actual fermentation is performed in a small-scale, which necessitates 1 day for preparing wort, and about 8 days to confirm the presence or absence of factors causing early flocculation in malt and barley according to the progress of fermentation. Further, as for oat before malting, about 7 days were necessary to prepare malt and then wort, thus about half a month was estimated to be necessary to confirm the presence or absence of factors causing early flocculation.

In order to shorten the period of the fermentation test to confirm the presence or absence of early flocculating property, a method for determining the presence or absence of factors causing early flocculation in the material oats, comprising the steps of adding an enzyme to a test material oat, performing enzyme treatment to the material oats, adding the obtained enzyme-treated substances or high-molecular weight fractions separated from the enzyme-treated substances to the synthesized wort to make a fermentation test material, and measuring turbidity of the fermentation test material 48 h later, was developed (Japanese Laid-Open Patent Application No. 10-179190). The period required to confirm the presence or absence of factors causing early flocculation has been significantly reduced by this method. However, a time period of 48 h for the fermentation test is still necessary and as the method is also a method using a fermentation test, thus, equipments for fermentation, etc. are necessary. Further, there is also a reference (Proc. Congr. Eur. Brew. Conv. 28: 397-406, 2001) describing a method wherein no fermentation test is performed, to confirm the presence or absence of early flocculating property, while a system for measuring accurately with quantativity has not been established yet. Therefore, a measuring method to confirm more easily the presence or absence of factors causing early flocculation in brewing materials, within a short time period, during brewing of fermented beverages and the like, was awaited.

Patent document 1: Japanese Laid-Open Patent Application No. 10-179190
Non-Patent document 1: Appl. Microbiol. Biotechnol 23:197-205, 2003Non-Patent document 2: K. Morimoto, et. Al., Rept. Res. Lab. Kirin Brewery Co., Ltd., 18, 63, 1975
Non-Patent document 3: J. Inst. Brew., 97, 359-366, 1991 Non-Patent document 4: Journal of Japan Society for Bioscience, Biotechnology and Agrochemistry, 71, 381, 1997
Non-Patent document 5: Proc. Congr. Eur. Brew. Conv. 28: 397-406, 2001

### Disclosure of the Invention

### Object to be solved by the present invention

The object of the present invention is to provide a method for quickly measuring factors causing early flocculation of yeast contained in brewing materials used for manufacturing fermented malt beverages such as beer, HAPPOSHU or whiskey, and a method for quickly determining early flocculating property of yeast in brewing materials, by using thereof. Particularly, it is to provide a method for measuring factors causing early flocculation of yeast contained in brewing materials, in a short time period and easily, without processing fermentation process as in the conventional methods.

### Means to solve the object

The present inventors have made a keen study to solve the above object. Thus, they prepared yeast at late logarithmic growth phase or thereafter, and mixed and suspended the yeast with water-extracted high-molecular weight fractions from test material sample containing oat and malt in buffer solution, and measured the precipitation level of the mixed and suspended yeast. As a result, they found out that it is possible to measure factors causing early flocculation of yeast contained in brewing materials, in a quite short time period, without performing fermentation process as in conventional methods, and have thus completed the present invention. In the method for measuring factors causing early flocculation of yeast of the present invention, it is necessary to culture yeast to prepare and use yeast at late logarithmic growth phase or thereafter, while the yeast can be cultured previously and cryopreserved, and thus, quick action for measuring factors causing early flocculation can be taken.

Moreover, it is necessary to prepare a water-extracted high-molecular weight fraction of the test material sample, while the fractions can be readily prepared by a method for separating a high-molecular weight fraction such as ethanol precipitation, dialysis, ultrafiltration or gel filtration following the water extraction of brewing materials. Further, according to the method of the present invention, when measuring the precipitation level of yeast, it is possible to measure it accurately by a simple and easy means such as measuring method of optical density by using OD600. Thus, the measuring method of the present invention as a whole can be a method for measuring factors causing early flocculation of yeast, which can be performed easily and quickly. The method for measuring factors causing early flocculation of the present invention can be applied to measure factors causing early flocculation of any brewing materials, not only barley or malt used for brewing, but all other cereal materials, or extracts.

In other words, the present invention relates to: a method for quickly measuring factors causing early flocculation of yeast contained in brewing materials, comprising the following steps:
1) a step for preparing yeast at late logarithmic growth phase or thereafter;
2) a step for preparing a water extracted-high-molecular weight fraction of a test material sample;
3) a step for mixing and suspending the yeast prepared in step 1) with the high-molecular weight fraction prepared in step 2), in a buffer solution; and
4) a step for measuring a precipitation level of the yeast mixed and suspended in the above step 3) ("1"); the method for quickly measuring factors causing early flocculation of yeast contained in brewing materials according to "1", wherein the yeast prepared in step 1) is obtained by culturing yeast, and collecting yeast at late logarithmic growth phase or thereafter, or by further cryopreserving the collected yeast ("2"); the method for quickly measuring factors causing early flocculation of yeast contained in brewing materials according to "2", wherein the collected yeast is washed with EDTA ("3"); the method for quickly measuring early flocculation factors of yeast contained in brewing materials according to any one of "1" to "3" , wherein the high-molecular weight fraction of step 2) is a high-molecular weight fraction prepared by ethanol precipitation of a water extraction solution of the test material sample, or a high-molecular weight fraction obtained by separating a water extraction solution of the test material sample by dialysis, ultrafiltration or gel filtration ("4"); the method for quickly measuring factors causing early flocculation of yeast contained in brewing materials according to any one of "1" to "4", wherein the high-molecular weight fraction of step 2) is a high-molecular fraction prepared from a wort of the test material sample ("5"); the method for quickly measuring factors causing early flocculation of yeast contained in brewing materials according to any one of "1" to "4", wherein the test material sample is treated with enzyme during extraction, when preparing the water-extracted high-molecular weight fraction of step 2) ("6"); the method for quickly measuring factors causing early flocculation of yeast contained in brewing materials according to any one of "1" to "6", wherein acetate buffer containing CaCl₂ is used as buffer solution of step 3) ("7"); the method for quickly measuring factors causing early flocculation of yeast contained in brewing materials according to any one of "1" to "7", wherein the precipitation level of yeast of step 4) is measured by measuring optical density by using OD600 ("8"); the method for quickly measuring factors causing early flocculation of yeast contained in brewing materials according to "8" , wherein the test system mixed and suspended in step 3) is allowed to stand for 15 min or more, which system is further mixed and suspended to measure serial change of optical density by using OD600, when measuring the precipitation level of yeast in step 4) ("9"); the method for quickly measuring factors causing early flocculation of yeast contained in brewing materials according to "9", wherein the test system mixed and suspended in step 3) is allowed to stand for 30 min or more ("10"); the method for quickly measuring factors causing early flocculation of yeast contained in brewing materials according to any one of "8" to "10" , wherein the relative precipitation level of yeast of the test material sample is measured by using water or a non-early flocculating material as a sample instead of the test material sample of step 2), and the measured serial change of optical density of the sample by using OD600 is used as a control to compare with the measured optical density by using OD600 of the test material sample, when measuring the precipitation level of yeast in step 4) ("11"); the method for quickly measuring factors causing early flocculation of yeast contained in brewing materials according to "8" , wherein the precipitation level of yeast is measured by measuring the optical density by using OD600 after allowing to stand still for 2 min or more, instead of measuring the serial change of optical density of the test sample by using OD600 after allowing the test system mixed and precipitated in step 3) to stand for 15 min or more, which system is further mixed and suspended, when measuring the precipitation level of yeast in step 4) ("12"); the method for quickly measuring factors causing early flocculation of yeast contained in brewing materials according to any one of "1" to "12", wherein the test material sample is barley, malt or malting barley ("13"); the method for quickly measuring factors causing early flocculation of yeast contained in brewing materials according to any one of "1" to "13", wherein the water-extracted high-molecular weight fraction of the test material sample is a high-molecular weight fraction of an extraction solution wherein ground malt materials are extracted with water for 30 sec or more, or a high-molecular weight fraction of an extraction solution wherein barley ground materials or malting barley-ground materials are extracted with water for 15 min or more ("14"); a method for quickly determining early flocculation property of yeast contained in brewing materials, using the method for quickly measuring factors causing early flocculation of yeast contained in brewing materials according to any one of "1" to "14" ("15").
Further, the present invention relates to: a method for manufacturing malt by using a method for quickly determining early flocculation property of yeast in brewing materials, wherein the malt manufacturing process is controlled by determining early flocculating property of malt as raw material, malt under manufacture, or malt, by using the method for quickly measuring factors causing early flocculation of yeast contained in brewing materials according to any one of "1" to "14" ("16"); a method for manufacturing fermented alcoholic beverages, wherein the brewing materials to be used are selected and adjusted by using the method for quickly measuring factors causing early flocculation factors contained in brewing materials according to any one of "1" to "14" by determining the early flocculating property of the brewing materials ("17"). Effect of the present invention

The early flocculating phenomenon of yeast occurring during the fermentation process of brewing fermented malt beverages such as beer, HAPPOSHU or whiskey, affects seriously the quality of the products. Therefore, when brewing fermented malt beverages and the like, it is very important to measure the presence or absence of factors causing early flocculation in the materials used for brewing, to determine the early flocculating property of the material, and to reflect these results for selecting or preparing the materials. According to the method for quickly measuring factors causing early flocculation of yeast of the present invention, it is possible to measure the presence or absence of factors causing early flocculation of materials to be used in brewing, quickly and accurately with a simple means, without depending on a fermentation method as in conventional methods, when brewing fermented malt beverages and the like. For example, it is possible to measure/determined the presence or absence of factors causing early flocculation in brewing materials such as barley or malt, used in brewing, accurately and with good reproducibility within a short time period of about 3 hours. Therefore, it is very useful as a practical method for measuring/determining the early flocculating property of brewing materials, used in brewing fermented malt beverages and the like.

Further, the method for quick measurement of the present invention can be applied for measuring factors causing early flocculation of malt or barley before malting, as well as barley immediately after harvesting, or malting barley, or other cereals used in brewing or extracts, and it is a method that can be widely used for determining the presence or absence of factors causing early flocculation in the brewing materials, as an easy, quick and accurate method. Moreover, as it is possible to perform the measurement with a minute amount of sample, the measuring method of the present invention can be used readily for brewing fermented malt beverages and the like, and to be provided as an accurate and practical measurement/determination method. Furthermore, according to the method for measuring factors causing early flocculation of the present invention, the measurement of factors causing early flocculation of each material can be controlled accurately so that it is possible to determine the early flocculating property for each material and to measure the activity of even a small amount of factors causing early flocculation. Thus, it is possible to measure factors causing early flocculation of each divided fraction in each brewing material. Thus, it can be also used effectively for purification or identification of the factors causing early flocculation.

### Best Mode of Carrying Out the Invention

The method for quickly measuring factors causing early flocculation of yeast contained in brewing materials of the present invention comprises the following steps.
1) a step for preparing yeast at late logarithmic growth phase or thereafter;
2) a step for preparing water extracted-high-molecular weight fraction of test material sample;
3) a step for mixing and suspending the yeast prepared in step 1) with the high-molecular weight fraction prepared in step 2), in a buffer solution; and
4) a step for measuring a precipitation level of yeast mixed and suspended in the above step 3).

In step 1) of the measuring method of the present invention, to culture yeast at late logarithmic growth phase or thereafter, yeast used for brewing is cultured in a medium for yeast commonly used, a proliferation curve of yeast is drawn, and yeasts that have attained the late logarithmic growth phase or thereafter (generally 4 to 5 days of culture at a temperature of 8°C) are separated by centrifugation, etc. and collected. The collected yeasts are preferably washed with a chelate compound solution, for example, such as 0.1m EDTA. The collected yeasts can be prepared as yeast of step 1), by cryopreserving yeasts that have been cultured and collected previously, for example, similarly as yeasts cryopreseved at -80°C with 15% glycerol solution.

In step 2) of the measuring method of the present invention, to prepare a water-extracted high-molecular weight fraction of the test material sample, test material samples including barley or malt are ground according to need followed by water extraction or normal mashing. When performing water extraction of the test material samples, enzyme treatment can be performed by adding α-amirase, β-amirase, β-glucanase, protease, etc. to the test material samples, according to the method described in Japanese Laid-Open Patent Application No. 10-179190. When preparing a water-extracted high-molecular weight fraction of the test material sample, separation of high-molecular weight fraction from water extraction solution can be performed by ethanol precipitation method, for example by adding ethanol to water extraction solution at a final concentration of 50%, and collecting the precipitates by centrifugation, or by a method for fractionating and collecting by dialysis, ultrafiltration, gel filtration, etc.

In step 3) of the measuring method of the present invention, for mixing and suspending the yeasts prepared in step 1) and the high-molecular weight fraction prepared in step 2) in a buffer solution, for example, solution such as 50 mM acetate buffer (pH 4 - 4.5) - 0.1% CaCl₂ can be used as a buffer solution.

In step 4) of the measuring method of the present invention, for measuring the precipitation level of the yeasts mixed and precipitated in step 3), it can be performed by a measurement of optical density by using OD600. For measuring the optical density, it can be performed by allowing the test system mixed and suspended in step 3) to stand for 15 min or more, which system is further mixed and suspended to measure the serial change of optical density of the test system by using OD600. For measuring the optical density, in order to further clarify the precipitation level of the yeast, it is preferable to allow the test system mixed and suspended in step 3) to stand for 30 min or more.

For measuring the precipitation level of yeast in step 4) of the measuring method of the present invention, the relative precipitation level of yeast of the test material sample can be measured by using water or non-early flocculating materials as a sample, instead of using the test material sample of step 2), and using the seiral change of the optical density measured for the sample by using OD600 as a control, which is compared with the optical density measuring for the test material sample by using OD600. Further, when measuring the precipitation level of yeast in step 4), the precipitation level of yeast can be measured by measuring the optical density of the test system mixed and suspended in step 3) by using OD600, after allowing the test system to stand for 2 min or more, instead of measuring the serial change of optical density of the test system by using OD600 after allowing the test system to stand for 15 min or more which system is further mixed and suspended.

The measuring method of the present invention can be used advantageously as a method for measuring factors causing early flocculation of yeast readily, quickly as well as accurately for barley, malt or malting barley, that are used as brewing materials. Further, it can be similarly used for other cereals or extracts used for brewing, as a method for measuring factors causing early flocculation of yeast readily, quickly as well as accurately. The measuring method of factors causing early flocculation of yeast of the present invention enables the measurement of the presence or absence of factors causing early flocculation for each brewing materials, and thus the determination of early flocculating property for each brewing materials is possible. Factors causing early flocculation included in each brewing material, are extracted, in case of malt, as a high-molecular weight fraction of extraction solution of ground malt materials extracted with water for 30 sec or more, and in case of ground barley materials or malting barley, as a high-molecular weight fraction of extraction solution of ground materials extracted with water for 15 min or more. Therefore, by using the method of the present invention for the brewing materials for the high-molecular weight fraction, it is possible to determine the presence or absence of factors causing early flocculation

In the following, the present invention will be explained in more detail by referring to the examples, while the technical scope of the present invention will not be limited to these exemplifications.

### Example 1

### [Method of example]

### 1) Culture of yeast

The preculture solution cultured for 3 days in an YEPG medium (1% Yeast Extract, 2% Bacto peptone, 7.5% Maltose, 2.5% Glucose) at 20°C by a static culture, was added to an YEPG medium (normally, 100 ml of medium in 250 ml-volume medium bottle) cooled previously at 8°C, at a concentration of 1.5%, and cultured at 8°C by stirring with a stirrer. During the culture, OD600 was measured serially, and a proliferation curve of yeast was drawn. Yeasts at logarithmic growth phase, at late logarithmic growth phase when the proliferation curve starts to get horizontal (4 days after the initiation of the culture), and at an early stationary phase were used.

### 2) Preparation of yeast for test

Yeast which had been cultured, were collected by centrifugation at 3000 rpm, for 5 min (4°C). The collected yeasts were suspended and washed with cold water, and were similarly collected by centrifugation. The resultant was suspended and washed twice with cooled 100 mM EDTA (pH 8.0), then suspended and washed twice with cold water, and was suspended into 20 ml of cold water. Yeast solution was diluted to 1/200, and yeast concentration was measured by measuring OD600. Further, after washing with cold water twice, the resultant was suspended in 15% glycerol, and after similarly measuring yeast concentration by measuring OD600 of the yeast solution, was frozen at -80°C.

### 3) Preparation of fractions of ethanol precipitates of wort

10 g or 50 g of malt was grounded roughly, congress wort prepared at a mash concentration of 1: 6 (45°C: 30 min → 1°C / 1 min → 70°C: 60 min after, filtration) was transferred to a centrifugation-tube, and ethanol was added at a final concentration of 50%. Precipitates were collected by centrifugation at 8000 rpm for 10 min (4°C), and were suspended in hot water of 20 ml/50 g malt (4 ml/10 g malt). Suspension was transferred to a 50 ml-tube, which was filled up with water to 25 ml/50 g malt (5 ml/10 g malt), and insoluble matters were removed by centrifugation at 8000 rpm for 10 min (20°C). The supernatants were used as measurement samples, respectively.
As a control for measuring, fractions of ethanol precipitates of wort prepared similarly from malt not causing early flocculation (hereinafter referred to as non-early flocculating malt), and malt causing early flocculation (hereinafter referred to as early flocculating malt) were used.

### 4) Preparation of fractions of ethanol precipitates of an enzyme preparation solution from barley and malting barley

Enzyme preparation solution from barley, was prepared according to the method described in Japanese Laid-Open Patent Application No. 10-179190, by adding α-amirase, β-amirase, β-glucanase, and protease. Preparation of fractions of ethanol precipitates were performed similarly as the above 3). As a control for measuring, fractions of ethanol precipitates of enzyme preparation solution similarly prepared from non-early flocculating barley and early flocculating barley were used.

### 5) Preparation of fractions of ethanol precipitates of a water-extraction solution from malt and barley

5 g of malt or barley ground materials were taken into a tube, 30 ml of water was added, and was shaken for 30 sec, 5 min, 15 min, and 30 min, respectively. After shaking for a predetermined time, the mixture was centrifuged at 7000 rpm for 10 min, supernatants were collected, and then ethanol was added to a final concentration of 50%. Precipitates were collected by centrifugation at 8000 rpm for 10 min (4°C), and suspended with 2.5 ml of hot water. Insoluble matters were removed by centrifugation at 8000 rpm for 10 min (20°C), and supernatants were used as samples for measurement. As a control for measuring, fractions of ethanol precipitates of enzyme preparation solution, similarly prepared from non-early flocculating barley and malt, and from early flocculating barley and malt were used.

### 6) Purification of substances inducing early flocculation (factors causing early flocculation) in wort, and gel filtration fraction of purified early flocculation factor

Purification of factors causing early flocculation was performed as follows.
Factors were sequentially eluted from the collected yeast fermented with early flocculating wort, with water and 0.1 M, 0.5 M, 1 M of NaCl. 0.1 M, 0.5 M NaCl eluted fractions having early flocculating activity were fractionated with DEAE-Sephadex A25 chromatography. Active fractions were re-fractionated with DEAE-Sephadex A 25 chromatography. For active fractions obtained by re-fractionation, protein was removed by phenol/chroloform treatment, to obtain purified factors of early flocculation. To measure the molecular weight of purified factors causing early flocculation, gel filtration by BioGel P100 was performed. Total amount of sugar was measured by phenol-sulphate method, which is a common method.

### 7) Activity measurement

In a plastic cuvette (10 × 10 × 45 mm) to be used for spectrophotometer, the following was prepared.
100 mM sodium acetate (pH 4.8): 1.5 ml
Various samples: 0.4 ml
50% calcium chloride: 6 µl
Yeast solution: calculated to be 3 from the OD600 level measured in 2)
Water: adjusted to be a total volume of 3 ml
The upper part of the cuvette was sealed with a parafilm, and after agitating up and down for a determined number of time (usually 40 times), allowed to rest for 5 min, 15 min, 30 min, 60 min at room temperature. After having been rested for a determined period, the precipitated flocculated yeast were dispersed by flipping with nails, and all the samples used for measurement were mixed gently up and down for a determined number of times (usually 4 times), simultaneously, and the turbidity was measured every 1 min for 5 min, or 2 min after by using OD 600. The OD600 level measured 2 min after is shown as a ratio to OD600 level (blank) when water was added instead of sample, which was made as 1.

### Example 2

### (Activity measurement results from the difference of proliferation stage of yeast)

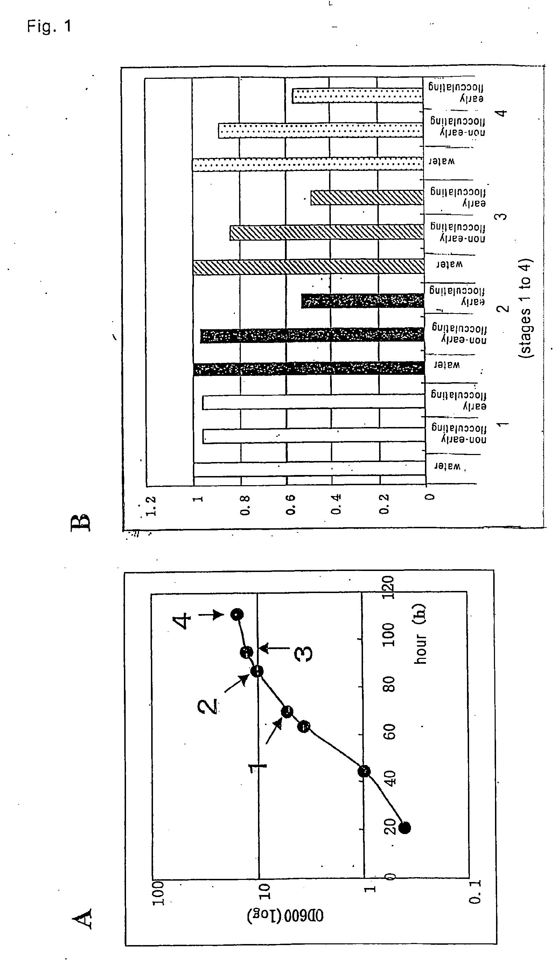

Activity measurement was performed by using the proliferation curve of yeast shown in the method 1) of Example 1, and fractions of ethanol precipitates of wort from non-early flocculating malt and early flocculating malt as a sample and by using yeast of different proliferation stages. The results are shown in Fig. 1. When it is described by the method 7) of Example 1, resting time after suspension was made to be 30 min. As it is clear from Fig. 1, it was revealed that by using yeast at late logarithmic growth phase or thereafter, when proliferation curve starts to get horizontal, early flocculating property can be estimated almost equally. On the other hand, when yeast at late logarithmic growth phase was used, it was revealed that early flocculating property could not be estimated. From the above results, the following experiments were performed by using yeast at late logarithmic growth phase.

### Example 3

### (Activity measurement result using yeast cryopreserved at -80°C in 15% glycerol solution)

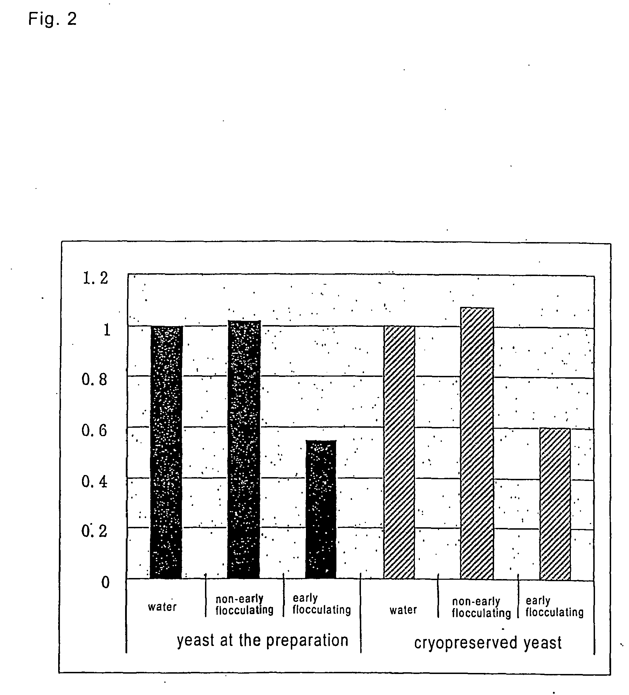

Yeast cryopreserved in 15% glycerol solution shown in the method 2) of Example 1 were melted, and activity was measured by using fractions of ethanol precipitates of wort from non-early flocculating malt or early flocculating malt as a sample. The results are shown in Fig. 2. When it is described by the method 7) of Example 1, resting time after suspension was made to be 30 min. As it is clear from Fig. 2, it has been revealed that yeast used for measurement gave similar results as that of yeast prepared just after culture, by cryopreserving with 15% glycerol.

### Example 4

### (Results from the difference of resting time at the time of activity measurement)

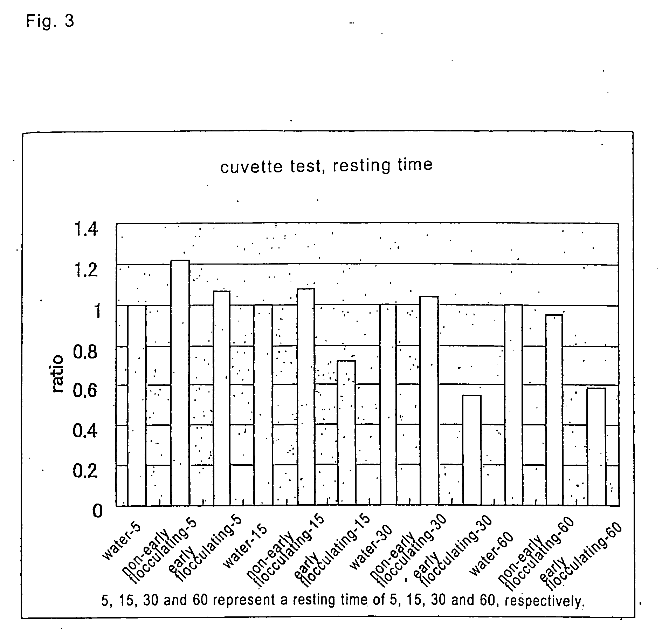

By using fractions of ethanol precipitates of wort from normal malt and early flocculating malt, comparison of activity measurement results caused by the difference of resting time after mixing and suspending according to the method 7) of Example 1 are shown in Fig. 3. As it is clear from Fig. 3, it has been revealed that with a resting time of 30 min or more, stable estimation of early flocculating property can be performed. It was also revealed that early flocculating property could be measured at a certain level with a resting time of 15 min. From the above results, resting time after mixing and suspending when measuring activity for the rest of the experiments was set to be 30 min.

### Example 5

### (Activity measurement using wort from malt)

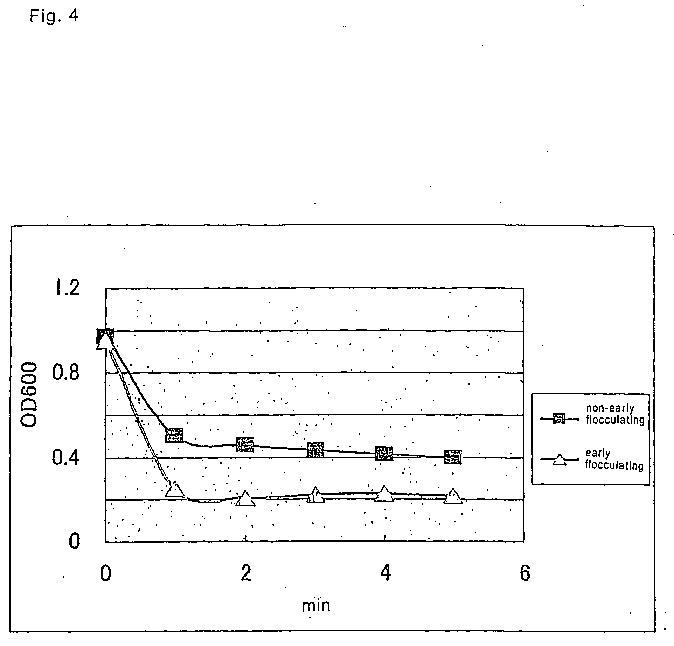

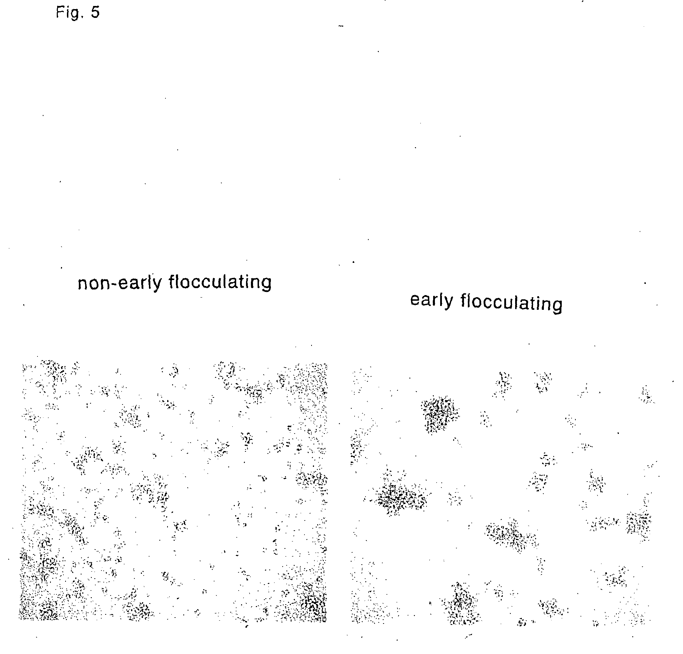

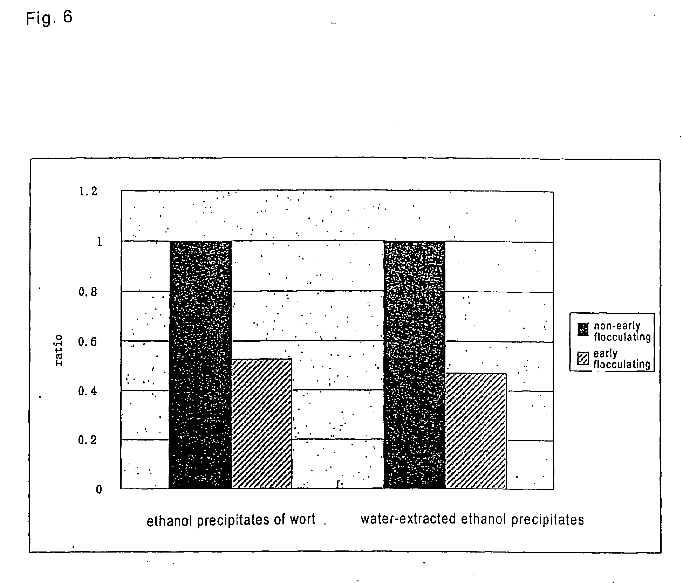

Serial change of turbidity by using OD600, according to the method 7) of Example 1, by using fractions of ethanol precipitates of wort from non-early flocculating malt and early flocculating malt, is shown in Fig. 4. Ethanol precipitates of early-flocculating wort added-test (early flocculating test) showed clearly early reduction of turbidity by yeast precipitation, compared to non-early flocculating test. Results of microscope observation of yeast precipitated by cuvette test, are shown in Fig. 5. Yeasts are in large cluster in early flocculating test compared to non-early flocculating test, thus supporting that the precipitation speed is faster. Precipitation was terminated 2 min later in both regions, and it was revealed that the OD600 level was maintained at a constant level until at least up to 5 min. From this result, it was revealed that measurement was possible by using the OD600 level that has become constant 2 to 5 min after mixing. The results of OD600 level in early flocculating test 2 min after mixing obtained as a ratio to the level in non-early flocculating test are shown in Fig. 6. From this result, it was revealed that early flocculating property could be determined from the measurement of 2 min after mixing.

### Example 6

### (Activity measurement using enzyme treatment solution from barley)

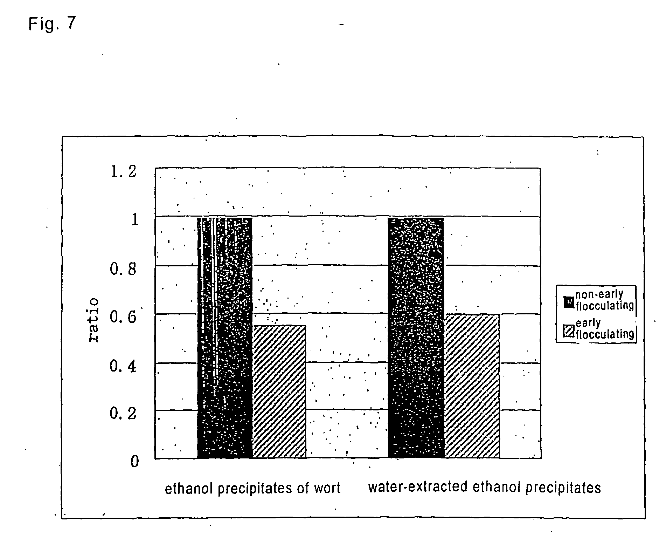

The turbidity of the enzyme treating solution from non-early flocculating barely and early flocculating barley measured by using the OD600 of 2 min after that described in the method 7) of Example 1 by using fractions of ethanol precipitates were shown as a ratio of early flocculating test to non-early flocculating test, and the results are shown in Fig. 7. From this result, it was revealed that early flocculating property could be determined from the measurement at 2 min after mixing.

### Example 7

### (Activity measurement using water extraction solution of malt and barley)

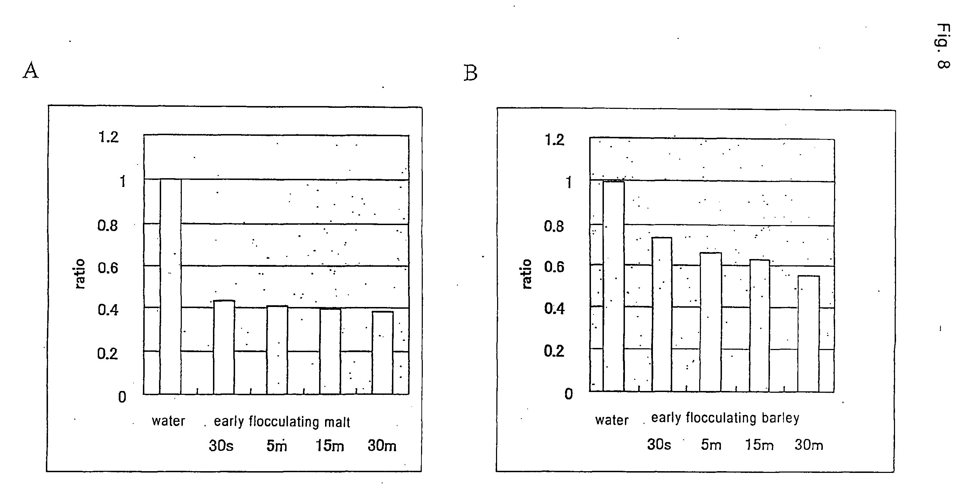

Ground materials of early flocculating malt and early flocculating barley were penetrated into water for 30 sec, 5, 15, 30 min, respectively. By using fractions of ethanol precipitates of the extraction solution as a sample, turbidity by using OD600 2 min after that described in the method 7) of Example 1 was obtained as a ratio to a blank turbidity, and the results are shown in Fig. 8. Further, ground materials of non-early flocculating malt and early flocculating malt, and non-early flocculating barley and early-flocculating barley were penetrated into water for 30 min, respectively, and ethanol precipitation fractions of the extraction solution were used as a sample, and turbidity by using OD600 2 min after that described in the method 7) of Example 7) are shown in Figs. 6 and 7 shown as a ratio of early flocculating test to non-early flocculating test. From these results, in fractions of the water-extracted ethanol precipitates of malt, the majority was extracted in 30 sec, and almost whole amount was extracted for 5 min or more, thus, it was revealed that the early flocculating property could be measured by an extraction of 5 min or more. Further, in the fraction of precipitates of water-extracted ethanol from ground barley material, the majority was extracted in 15 min and almost whole amount was extracted in 30 min or more, it was revealed that the early flocculating property could be measured by an extraction of 30 min or more.

### Example 8

### (Activity measurement by using enzyme treatment solution of sample during malting)

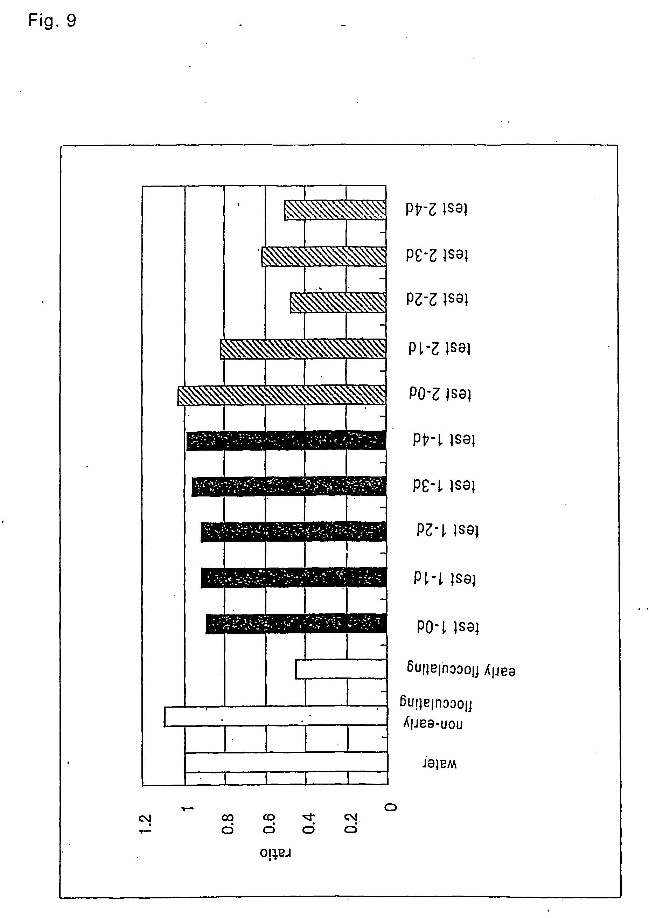

Sampling was performed every other day, from immediately after steeping of malting region of early-flocculation malting, and non-early flocculation malting, until up to day 4, and these samples were dried, ground and extracted with water for 30 min. The measurement results by the present method using ethanol-precipitated fractions of the extraction solution are shown in Fig. 9. In the non-early flocculation malting test, activity was not observed during the 4 days, and it was shown that factors causing early flocculation were not generated. On the other hand, in the early-flocculating malting test, activity was confirmed on day 1 of germination, and on day 2, activity comparable with that of finished malt, that is, generation of factors causing early flocculation was observed. From the above, it was shown that the present method was also useful as a system to monitor the level of factors causing early flocculation of samples during malting.

### Example 9

### (Result of activity measurement using fractions during purification process of factors causing early flocculation)

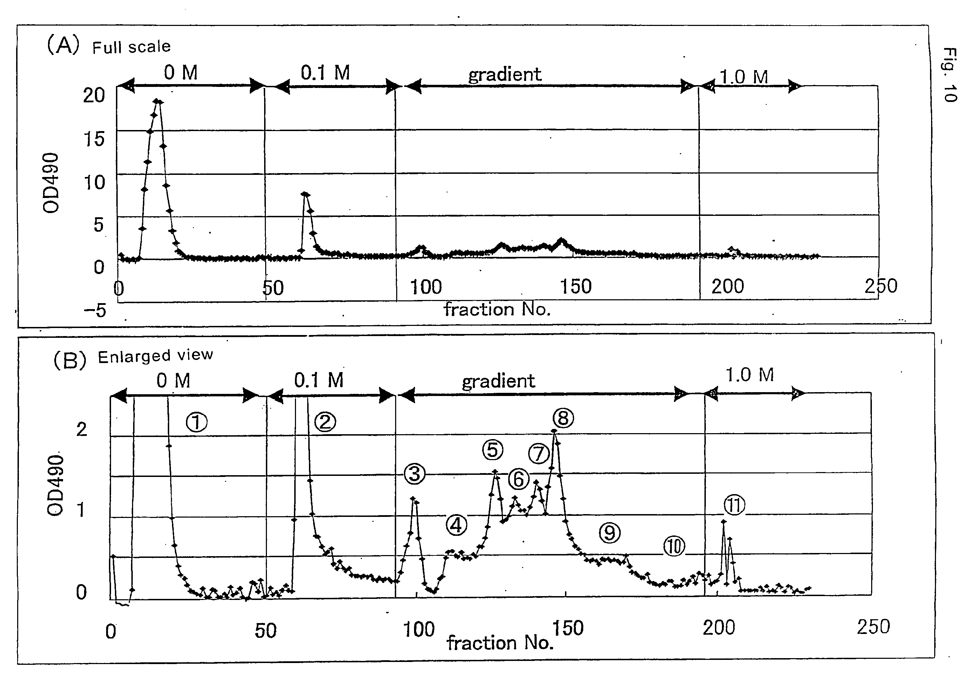

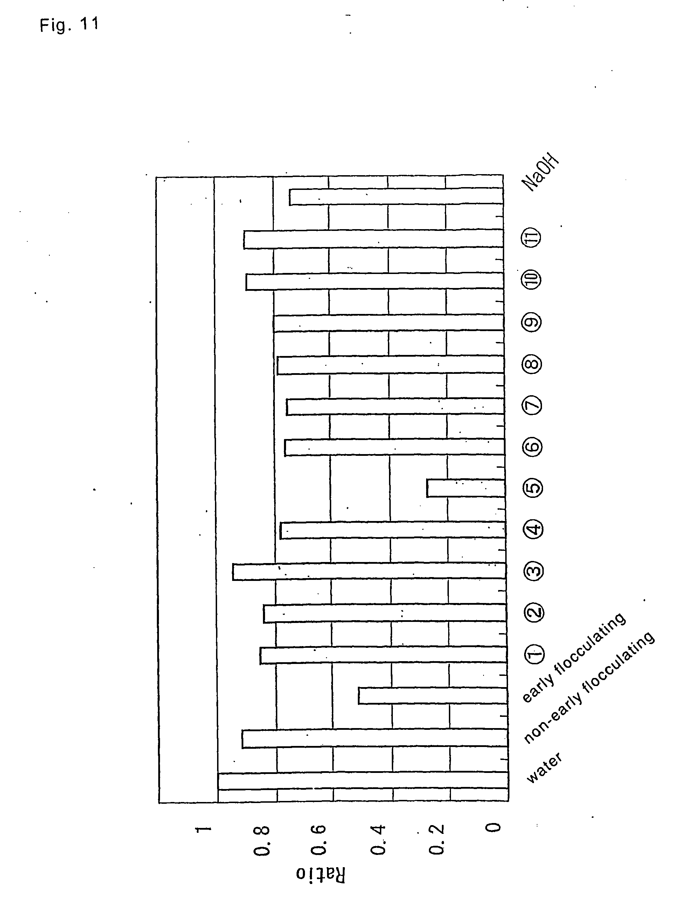

The fractionating result by the first DEAE-Sephadex A25 Chromatography during purification process of factors causing early flocculation described in the method 6) of Example 1 is shown in Fig. 10. By this fractionation, the factors were fractionated in 11 sugar peaks, including passing through (shown by 0M in figures).
Measurement results by the present method using the above 11 peaks as samples are shown in Fig. 11. Activity measurement was performed by using the sample in the reaction system described in the method 7) of Example 1 to 0.4 ml by adding fractions and water. As it is clear from Fig. 11, in the peaks shown in Fig. 10, activity was specifically observed for the peak described as "5" in Fig. 10. From the above results, it was revealed that the present method can be used in the purification process of factors causing early flocculation.

### Example 10

### (Activity measurement of fractions of gel filtration of purified-factors causing early flocculation)

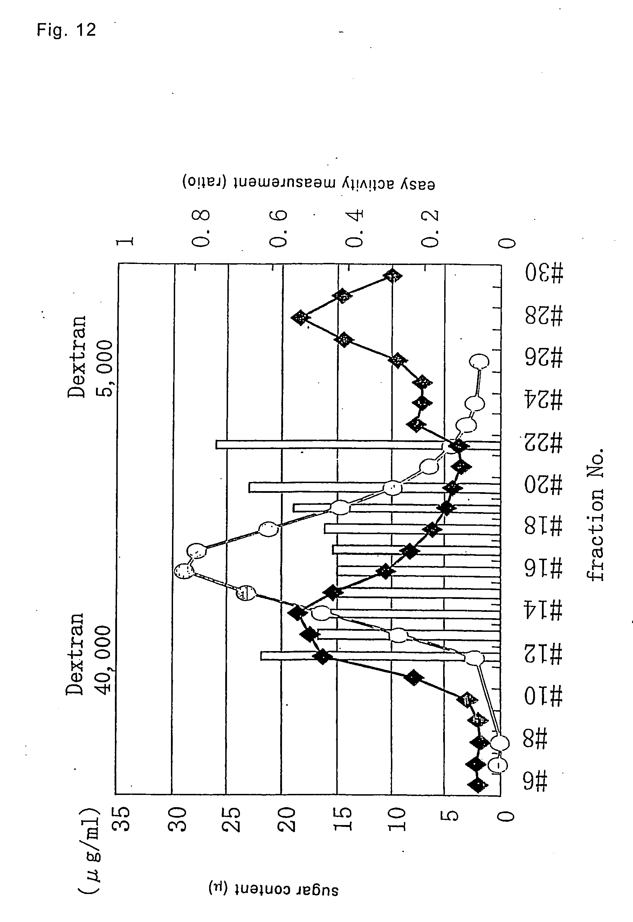

Results of fractionating factors causing early flocculation purified by the process described in the method 6) of Example 1, which were subjected to gel filtration by BioGel P100, and measurement results by the present method using the equivalent of 1 µg of sugar amount of each fraction as a sample, are shown in Fig. 12. As it is clear from Fig. 12, the provided sugar was fractionated as a single peak being symetric, and it was revealed to be purified polysaccharide. Early flocculating property measured with the present method was observed to the peak of purified sugar, and it was shown that purified substances were factors causing early flocculation. From the above results, it was shown that the present system was also a system that can measure early flocculating activity of factors causing early flocculation in a minute amount as 1 µg.

### Example 11

### (Measurement comparison of water-extracted malt solution with that using high-molecular weight fraction by ethanol precipitation as a sample) [Experiment method]

15 ml of 0.05 mM sodium acetate (pH 4.8) was added to 9 g of early flocculating malt and non-early flocculating malt, and mixed with a stirrer for 1 hour. After mixing, extraction solution was filtrated with paper filter, and further the filtrate was passed through a 0.45 µm filter, to make a measurement sample. By using 300 µl of sample solution (extraction solution equivalent to 0.18 g of malt), measurement was performed by the activity measurement method described in 7) of Example 1. For comparison, 90 µl (equivalent to 0.18 g of malt) of suspension of ethanol precipitation from early flocculating wort and non-early flocculating wort described in 3) of Example 1, and 400 µl (equivalent to 0.8 malt) as described in 7) were used as a sample to measure activity simultaneously.

### (Results)

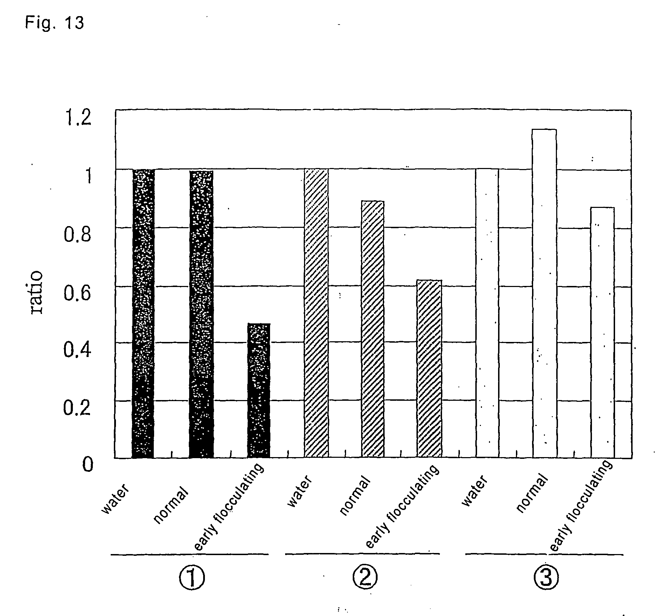

By the above method, water extraction solution and high-molecular weight fraction by ethanol precipitation by using early flocculating malt and non-early flocculating malt were subjected as samples to measure the activity, and the results are shown in Fig. 13. When water extraction equivalent to 0.18 g of malt was used as a sample, almost no difference was observed for the measurement level between the control test (water added) or non-early flocculating test (3 (circled) in Fig. 3). On the other hand, when wort ethanol precipitation was used as a sample, in the region where the equivalent of 0.18 g malt was added, turbidity was low in early flocculation test compared to that in control test (water added) or non-early flocculating region, and it was shown that early flocculating activity could be measured at a certain level (2 (circled) in Fig. 13). Further, in the region where equivalent of 0.8 g malt was added, the difference of turbidity was more significant (1 in Fig. 13). From the above results, it was revealed that the accuracy of measurement increases when extraction sample are high-molecular weight fractions obtained by ethanol precipitation as a sample, and that the accuracy of measurement further increases when a sample equivalent to 0.8 g malt in amount was used, compared to when a sample of equivalent of 0.18 g of malt is used.

### Example 12

### (Investigation of the optimum concentration of a sodium acetate buffer to be used for activity measurement) [Experiment method]

By using sodium acetate at a final concentration of 0.05, 0.5, 5, 50, 500 mM (pH4.8), by the method shown in 7) of Example 1, suspension of ethanol precipitation from early flocculating malt and non-early flocculating malt was used to measure the activity.

### (Results)

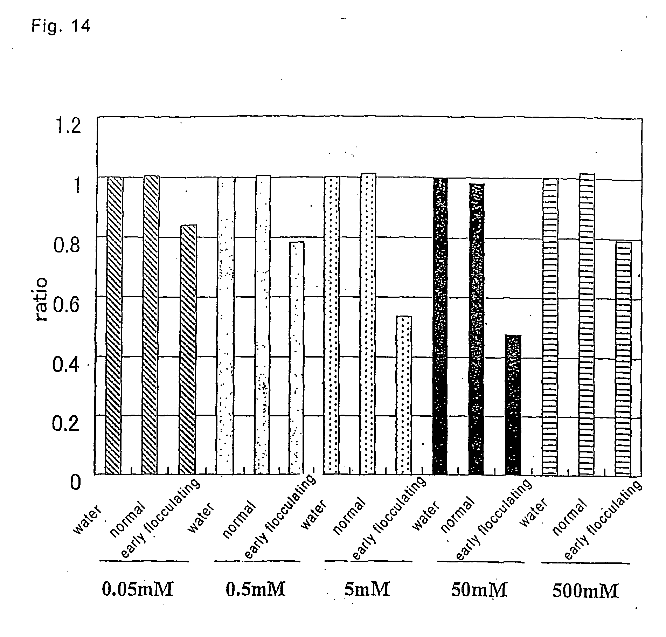

Measurement results of activity are shown in Fig. 14. As it is clear by this result, the measurement results depend on the concentration of sodium acetate (pH4.8) to be added. It was revealed that in the 50 mM buffer region, the difference of measurement level of early flocculation and non-flocculation was the largest, and that the most accurate measurement was performed in that region.

### Brief Description of Drawings

[Fig. 1] It is a figure showing the relation between the yeast culture stages and measurement results of early flocculating activity in the Examples of the present invention.
[Fig. 2] It is a figure showing the results comparing the early flocculating activity when yeast at the culture preparation is used, with that of when cryopreserved yeast is used, in the Examples of the present invention.
[Fig. 3] It is a figure showing the measurement results of early flocculating activity according to the resting time after mixing and suspending when measuring early flocculation activity in the Examples of the present invention.
[Fig. 4] It is a figure showing the serial turbidity change in the test using early flocculating wort and non-early flocculating wort in the Examples of the present invention.
[Fig. 5] It is a figure showing the results of photography of yeast precipitated in the cuvette, in the test using early flocculating wort and non-early flocculating wort, in the Examples of the present invention.
[Fig. 6] It is a figure showing the results of investigation of early flocculation activity when ethanol precipitation was used for early flocculating malt and non-early flocculating malt, in the Examples of the present invention.
[Fig. 7] It is a figure showing the results of investigation of early flocculation activity when ethanol precipitates was used for early flocculating barley and non-early flocculating barley, in the Examples of the present invention.
[Fig. 8] It is a picture showing the results of the relationship of water extraction time and factors causing early flocculation, for early flocculating malt and early flocculating barley ground material, in the Examples of the present invention.
[Fig. 9] It is a picture showing the results of test measuring early flocculation activity by using sample during malting, for early flocculating malt and non-early flocculating malt, in the Examples of the present invention.
[Fig. 10] It is a picture showing each peak of the result of fractionating the wort by using DEAE-Sephadex A25 Chromatography, in the Examples of the present invention. [Fig. 11] It is a picture showing the results of the test of early flocculating activity for each peak of the results fractionating the wort by using DEAE-Sephadex A25 Chromatography, in the Examples of the present invention. [Fig. 12] It is a picture showing the measurement results of fractionating factors causing early flocculation obtained by fractionating the wort by using DEAE-Sephadex A25 chromatography and further performing gel filtration by using BioGel p100, and of early flocculating activity by using the equivalent of 1 µg of sugar level of the fraction as a sample, in the Examples of the present invention.
[Fig. 13] It is a picture showing the comparison of the measurement results using the water extraction solution of malt and a high-molecular weight fraction by ethanol precipitation as samples, in the Example of the present invention.
[Fig. 14] It is a picture showing the results of investigating the optimal concentration of sodium acetate buffer used for activity measurement, in the Example of the present invention.

## Claims

1. A method for quickly measuring factors causing early flocculation of yeast contained in brewing materials, comprising the following steps:
1) a step for preparing yeast at late logarithmic growth phase or thereafter;
2) a step for preparing a water extracted-high-molecular weight fraction of a test material sample;
3) a step for mixing and suspending the yeast prepared in step 1) with the high-molecular weight fraction prepared in step 2), in a buffer solution; and
4) a step for measuring a precipitation level of the yeast mixed and suspended in the above step 3).

2. The method for quickly measuring factors causing early flocculation of yeast contained in brewing materials according to claim 1, wherein the yeast prepared in step 1) is obtained by culturing yeast, and collecting yeast at late logarithmic growth phase or thereafter, or by further cryopreserving the collected yeast.

3. The method for quickly measuring factors causing early flocculation of yeast contained in brewing materials according to claim 2, wherein the collected yeast is washed with EDTA.

4. The method for quickly measuring early flocculation factors of yeast contained in brewing materials according to any one of claims 1 to 3, wherein the high-molecular weight fraction of step 2) is a high-molecular weight fraction prepared by ethanol precipitation of a water extraction solution of the test material sample, or a high-molecular weight fraction obtained by separating a water extraction solution of the test material sample by dialysis, ultrafiltration or gel filtration.

5. The method for quickly measuring factors causing early flocculation of yeast contained in brewing materials according to any one of claims 1 to 4, wherein the high-molecular weight fraction of step 2) is a high-molecular weight fraction prepared from a wort of the test material sample.

6. The method for quickly measuring factors causing early flocculation of yeast contained in brewing materials according to any one of claims 1 to 4, wherein the test material sample is treated with enzyme during extraction, when preparing the water-extracted high-molecular weight fraction of step 2).

7. The method for quickly measuring factors causing early flocculation of yeast contained in brewing materials according to any one of claims 1 to 6, wherein acetate buffer containing CaCl₂ is used as buffer solution of step 3).

8. The method for quickly measuring factors causing early flocculation of yeast contained in brewing materials according to any one of claims 1 to 7, wherein the precipitation level of yeast of step 4) is measured by measuring optical density by using OD600.

9. The method for quickly measuring factors causing early flocculation of yeast contained in brewing materials according to claim 8, wherein the test system mixed and suspended in step 3) is allowed to stand for 15 min or more, which system is further mixed and suspended to measure the serial change of optical density by using OD600, when measuring the precipitation level of yeast in step 4).

10. The method for quickly measuring factors causing early flocculation of yeast contained in brewing materials according to claim 9, wherein the test system mixed and suspended in step 3) is allowed to stand for 30 min or more.

11. The method for quickly measuring factors causing early flocculation of yeast contained in brewing materials according to any one of claims 8 to 10, wherein the relative precipitation level of yeast of the test material sample is measured by using water or a non-early flocculating material as a sample instead of the test material sample of step 2),and the measured serial change of optical density of the sample by using OD600 is used as a control to compare with the measured optical density by using OD600 of the test material sample, when measuring the precipitation level of yeast in step 4).

12. The method for quickly measuring factors causing early flocculation of yeast contained in brewing materials according to claim 8, wherein the precipitation level of yeast is measured by measuring the optical density by using OD600 after allowing to stand still for 2 min or more, instead of measuring the serial change of optical density of the test sample by using OD600 after allowing the test system mixed and precipitated in step 3) to stand for 15 min or more, which system is further mixed and suspended, when measuring the precipitation level of yeast in step 4).

13. The method for quickly measuring factors causing early flocculation of yeast contained in brewing materials according to any one of claims 1 to 12, wherein the test material sample is barley, malt or malting barley.

14. The method for quickly measuring factors causing early flocculation of yeast contained in brewing materials according to any one of claims 1 to 13, wherein the water-extracted high-molecular weight fraction of the test material sample is a high-molecular weight fraction of an extraction solution wherein ground malt materials are extracted with water for 30 sec or more, or a high-molecular weight fraction of an extraction solution wherein barley ground materials or malting barley-ground materials are extracted with water for 15 min or more.

15. A method for quickly determining early flocculation property of yeast contained in brewing materials, using the method for quickly measuring factors causing early flocculation of yeast contained in brewing materials according to any one of claims 1 to 14.

16. A method for manufacturing malt by using a method for quickly determining early flocculation property of yeast in brewing materials, wherein the malt manufacturing process is controlled by determining early flocculating property of malt as raw material, malt under manufacture, or malt, by using the method for quickly measuring factors causing early flocculation of yeast contained in brewing materials according to any one of claims 1 to 14.

17. A method for manufacturing fermented alcoholic beverages, wherein the brewing materials to be used are selected and adjusted by using the method for quickly measuring factors causing early flocculation factors contained in brewing materials according to any one of claims 1 to 14 by determining the early flocculating property of the brewing materials.
